# EUROPÄISCHE PATENTSCHRIFT

(11) **EP 2 793 792 B1**
(45) Veröffentlichungstag und Bekanntmachung des Hinweises auf die Patenterteilung: **06.04.2016**
(21) Anmeldenummer: 12812225.6
(22) Anmeldetag: 19.12.2012
(51) Int. Cl.: A61G 5/10, A61G 5/12, B62B 7/00, B62B 9/10, B60N 2/28, B60N 2/26

(54) **KINDERSITZVORRICHTUNG FÜR EIN KIND UND KINDERWAGEN**
CHILD SEAT DEVICE FOR A CHILD AND STROLLER
DISPOSITIF DE SIÈGE ENFANT POUR UN ENFANT ET POUSSETTE

(30) Priorität: 20.12.2011 DE 102011089192
(43) Veröffentlichungstag der Anmeldung: 29.10.2014
(73) Patentinhaber: Serdyuk, Valentyn, 65058 Odessa (UA)
(72) Erfinder: SERDYUK, Valentyn, Odessa 65058 (UA)
(74) Vertreter: Isarpatent
(86) Internationale Anmeldenummer: PCT/EP2012/076120
(87) Internationale Veröffentlichungsnummer: WO 2013/092690

(56) Entgegenhaltungen:
- EP-A1- 0 109 572
- EP-A2- 0 311 993
- WO-A1-01/43685
- DE-T2- 69 306 643
- US-A- 4 753 482
- US-A- 5 758 926

## Beschreibung

### GEBIET DER ERFINDUNG

Die vorliegende Erfindung betrifft eine Kindersitzvorrichtung für ein Kind zur Ausrichtung der Wirbelsäule des Kindes und zur Vorbeugung und Behandlung (Skoliose) einer Wirbelsäulenverkrümmung des Kindes. Die Kindersitzvorrichtung kann hierbei an weiteren Einrichtungen, wie beispielsweise einem Autositz usw., befestigt werden zum Transport eines Kindes beispielsweise in einem Fahrzeug. Die vorliegende Erfindung betrifft ferner einen Kinderwagen.

### TECHNISCHER HINTERGRUND

Kinderwagen sind allgemein bekannt, sodass auf deren Aufbau und Funktionsweise nicht näher eingegangen werden muss. Ein Kinderwagen ist ein Hilfsmittel zum liegenden oder sitzenden Transport von Säuglingen und Kleinkindern. Bei einigen Kinderwägen lässt sich der Korb vom Gestell lösen und teilweise auch als Kinderautositz verwenden.

Ein Kindersitz, auch Rückhaltevorrichtung genannt, ist eine an die geringe Körpergröße von Kindern angepasste Sitzgelegenheit. Im Speziellen wird damit zumeist ein Sitz für den sicheren Transport von Kindern in Fahrzeugen bezeichnet, der über Befestigungssysteme für den Sitz und Rückhaltesysteme für das Kind verfügt oder der zumindest die Sitzposition des Kindes so erhöht, dass der für den Sitzplatz vorgesehene fahrzeugeigene Sicherheitsgurt nicht am Hals entlang, sondern über die Schulter verläuft.

In der WO 01/43685 ist eine mehrteilige anatomisch an den Patienten angepasste ortsunabhängige Sitzschale offenbart. Sie besteht aus einer auf den Rollstuhl auflegbaren oder festspannbaren Grundplatte, an deren Vorderkante eine Sitzfläche kippfähig angelenkt ist. Diese Sitzfläche ist im Gesäß als Teil der Rückenlehne bis zum Lumbalbereich hochgezogen und als Gesäßstützfläche ausgeformt.
Der obere Teil der Rückenlehne ist an dieser schwenkbar befestigt. Zugleich sind an der oberen Rückenlehne, gegenüber der Mittelachse und auf den Lumbalbereich bezogen, seitlich ausschwenkbare und elastisch abgestützte Thoraxpelotten angeordnet.

Aus der US 5,758,926 A ist ein einstellbares Sitzsystem bekannt, wobei das Sitzsystem einen Sitz und einen Stand für körperbehinderte Menschen aufweist. Der Sitz weist dabei eine Basis mit zwei Seitenteilen auf, sowie ein Rückenteil, das benachbart zu dem Sitz angeordnet ist. Das Sitzrückenteil weist zwei erste und zweite Thoraxstützen auf.

Die menschliche Wirbelsäule bildet eine Vielfalt von Bögen, die jeweils gegenüber angeordnet sind und sich somit ausbalancieren, um den Körper im Gleichgewicht zu halten. In der Sagittalebene bildet die Wirbelsäule vier physiologische Krümmungen aus, Nacken-Lordose, Brust-Kyphose, Lenden-Lordose und Steißbein-Kyphose. Abweichungen der Wirbelsäule in der Frontalebene nach rechts oder links werden als Skoliose bezeichnet. Bis ein Kind zu laufen beginnt, wird eine (gewisse) Skoliose als physiologisch angesehen und als skoliotische Haltung bezeichnet. Später allerdings kann die Muskulatur die skoliotische Deformation nicht mehr ausgleichen, bedingt durch ein gleichzeitiges Rotieren der Wirbel und die nachfolgende Minderentwicklung der Wirbelkörper, -bögen und -bänder, weshalb sie krankhaft wird.

Skoliosen werden klassifiziert nach ihrer Ursache und dem Entstehungszeitpunkt, nach der Lage ihrer Krümmungen und dem Krümmungsmuster, nach ihrem Ausmaß (Krümmungswinkeln und Rotationsgraden) und der Ausrichtung der Krümmungen (links, rechts). Es hat sich herausgestellt, dass die Skoliose im Kindesalter und in Zeiten starken Körperlängenwachstums entsteht und sich verschlechtert. Je schneller das Körperwachstum ist, desto schneller nehmen die Krümmungen zu. Dementsprechend zählen Skoliosen auch zu den Wachstumsdeformitäten.

Derzeit werden Skoliosen durch das Einsetzen eines Implantats oder durch das Tragen eines orthopädischen Korsetts behandelt. Das Einsetzen eines Implantats stellt jedoch einen erheblichen chirurgischen Eingriff dar, sodass dies für Kleinkinder nicht geeignet ist. Auch das Tragen eines orthopädischen Korsetts ist für Kleinkinder kein geeignetes Mittel, um Skoliosen zu behandeln. Insofern reduzieren sich diese derzeit bekannten Mittel zur Behandlung von Skoliosen auf die Anwendung bei Erwachsenen oder größeren Kindern. Bei Kleinkindern existieren derzeit keine geeigneten Mittel zur Behandlung von Skoliosen, obgleich bei diesen Kleinkindern aufgrund deren sehr großem Körperwachstums die Gefahr einer entstehenden Skoliose am größten ist.

Dies ist ein Zustand den es zu verbessern gilt.

### ZUSAMMENFASSUNG DER ERFINDUNG

Vor diesem Hintergrund liegt der vorliegenden Erfindung die Aufgabe zugrunde, eine Möglichkeit zur Vorbeugung und Behandlung einer Wirbelsäulenverkrümmung (Skoliose) bei Kleinkindern bereitzustellen.

Erfindungsgemäß wird diese Aufgabe durch eine Kindersitzvorrichtung mit den Merkmalen des Patentanspruchs 1 und/oder durch einen Kinderwagen mit den Merkmalen des Patentanspruchs 12 und/oder durch einen Kinderantositz mit den Merkmalen des Patentanspruchs 15 gelöst.

Die der vorliegenden Erfindung zugrunde liegende Idee besteht darin, eine Kindersitzvorrichtung bereitzustellen, bei welcher eigens vorgesehene Stützelemente vorhanden sind, welche von hinten nach vorne den Oberkörper des Kindes auf der Ebene der Achselhöhle einfassen und damit stützen, fixieren und ausrichten. Die erfindungsgemäße Kindersitzvorrichtung hat den besonderen Vorteil, dass das Körpergewicht des Kindes so auf den Stützelementen zum Ruhen kommt. Der Körper des Kindes wird aufgerichtet und erfährt vorzugsweise auch einen leichten Zug nach oben. Gleichzeitig wird eine Seitenbewegung des Kindes durch die Stützelemente beschränkt. Durch die erfindungsgemäße Kindersitzvorrichtung wird damit einer Wirbelsäulenverkrümmung vorgebeugt oder eine vorhandene Wirbelsäulenverkrümmung wird behandelt.

Vorteilhafte Ausgestaltungen und Weiterbildungen ergeben sich aus den weiteren Unteransprüchen sowie aus der Beschreibung unter Bezugnahme auf die Figuren der Zeichnung.

In einer besonders vorteilhaften Ausgestaltung sind die Stützelemente höhenverstellbar und/oder seitlich verstellbar ausgebildet. Dadurch können die Stützelemente sehr einfach an unterschiedlich große Kinder angepasst werden. Durch eine entsprechende Höheneinstellung wird auch ein gewünschter definierter Zug auf das Kind und insbesondere dessen Wirbelsäule ausgeübt, sodass das Kind möglichst aufrecht und nicht seitlich gebeugt in dem Sitz sitzt. Insbesondere kann durch eine entsprechende Höheneinstellung ein gewünschter definierter Zug auf das Kind, oberhalb der Brust des Kindes, ausgeübt werden. Aufrecht in diesem Kontext bezeichnet einen Zustand, bei dem die Wirbelsäule die eingangs erwähnte, S-Form aufweist, sodass weitestgehend keine Seitverbiegung der Wirbelsäule bei gleichzeitiger Verdrehung der Wirbel entsteht.

In einer weiteren vorteilhaften Ausgestaltung ist eine Verschlussvorrichtung vorgesehen, über welche die Stützelemente an ihrer Vorderseite, also auf der Seite der Brust des sitzenden Kindes, verschließbar ausgebildet sind. Die Verschlussvorrichtung kann zum Beispiel als Gurt mit Klippsverschluss, Klettverschluss, Schnalle oder dergleichen ausgebildet sein. Auf diese Weise kann auch auf eine Fixierung der Kindersitzvorrichtung mittels eines Gurts, welcher durch die Beine des Kindes verläuft, verzichtet werden. Zudem erhöht sich damit auch der Komfort für das Kind.

In einer vorteilhaften Ausgestaltung sind zumindest ein Stützelement und vorzugsweise beide Stützelemente gepolstert ausgebildet. Dadurch werden Druckstellen am Oberkörper des Kindes vermieden oder zumindest abgeschwächt.

Ebenfalls in einer bevorzugten Ausgestaltung sind zumindest ein Stützelement und vorzugsweise beide Stützelemente an deren Oberseite, auf der im bestimmungsgemäßen Gebrauch ein Arm des Kindes aufliegt, elastisch, abgerundet und/oder abgeflacht ausgebildet. Auch dies führt dazu, dass Druckstellen an den Oberarmen des Kindes vermieden oder zumindest abgeschwächt werden.

In einer vorteilhaften Ausgestaltung ist zumindest ein federelastisches Element vorgesehen, über welches ein Stützelement mit dem Sitzoberteil federelastisch gekoppelt ist. Das federelastische Element ist dazu vorgesehen, bei vertikaler und/oder horizontaler Belastung des Stützelementes ein federelastisches Nachgeben des Stützelementes zu ermöglichen. Durch diese Art des flexiblen Nachgebens erhöht sich der Komfort für das im Sitz sitzenden Kindes, ohne dass dessen Funktionalität im Hinblick auf die Vermeidung oder Behandlung der Wirbelsäulenverkrümmung verringert wird.

In einer vorteilhaften Ausgestaltung sind das Sitzoberteil und/oder das Sitzunterteil schwenkbar ausgebildet. Das Sitzoberteil und/oder das Sitzunterteil sind insbesondere in wenigstens einem vorbestimmten Winkel zu dem Sitzunterteil bzw. dem Sitzoberteil arretierbar ausgebildet. Diese schwenkbare Funktion hat den Vorteil, dass die Kindersitzvorrichtung sehr einfach an die Stellung einer Rückenlehne eines Kinderwagens, z.B. eines Buggys, angepasst werden kann, wenn die Kindersitzvorrichtung in dem Kinderwagen angeordnet oder angebracht wird.

In einer weiteren vorteilhaften Ausgestaltung ist das Sitzoberteil ausziehbar ausgebildet. Dadurch kann beispielsweise ein an dem Sitzoberteil befestigtes, gepolstertes Kopfteil sehr einfach an verschieden große Kinder angepasst und so der Komfort der Kindersitzvorrichtung für das Kind erhöht werden.

In einer vorteilhaften Ausgestaltung sind die Kindersitzvorrichtung und dabei insbesondere dessen Sitzoberteil, Sitzunterteil und Stützelemente derart dimensioniert und ausgebildet, um ein Kleinkind, insbesondere ein Kind in einem Alter von 1 bis 3 Jahren, aufzunehmen.

Eine besonders vorteilhafte Ausgestaltung sieht vor, dass die Kindersitzvorrichtung als ein Kinderautositz für ein Kraftfahrzeug ausgebildet ist. Die Kindersitzvorrichtung kann dabei als lösbarer Bestandteil in dem Kinderautositz angeordnet und/oder befestigt werden oder alternativ als integraler Bestandteil des Kinderautositzes ausgebildet sein.

Eine alternative, ebenfalls vorteilhafte Ausgestaltung des erfindungsgemäßen Kinderwagens sieht vor, dass die Kindersitzvorrichtung fester, also integraler Bestandteil des Kinderwagens ist. Alternativ wäre auch denkbar, dass die Kindersitzvorrichtung als lösbarer Bestandteil in den Kinderwagen einsetzbar ist und über eine Befestigungseinrichtung, beispielsweise mittels Gurten, Klipse und dergleichen, an einem Rahmen des Kinderwagens lösbar befestigbar ist.

In einer anderen erfindungsgemäßen Ausführungsform weisen Sitzoberteil und/oder das Sitzunterteil zumindest zwei, über wenigstens einen Querträger miteinander verbundene Längsträger auf. Dadurch kann eine besonders leichte und preisgünstige Kindersitzvorrichtung bereitgestellt werden. Gemäß einer weiteren Ausführungsform der Erfindung sind das Sitzoberteil und das Sitzunterteil über einen gemeinsamen Querträger fest oder um den Querträger schwenkbar verbunden. Eine feste Anbindung des Sitzoberteils und des Sitzunterteils an den gemeinsamen Querträger hat den Vorteil, dass die Kindersitzvorrichtung besonders preisgünstig in der Herstellung ist. Der jeweilige Längsträger des Sitzoberteils weist ein erstes Längsträgerteil und ein zweites Längsträgerteil auf, welches verschiebbar mit dem ersten Längsträgerteil verbunden ist. Vorzugsweise weist das Sitzoberteil und das Sitzunterteil jeweils wenigstens eine Abdeckung oder Polsterung auf.

Die obigen Ausgestaltungen und Weiterbildungen lassen sich, sofern sinnvoll, beliebig miteinander kombinieren. Weitere mögliche Ausgestaltungen, Weiterbildungen und Implementierungen der Erfindung umfassen auch nicht explizit genannte Kombinationen von zuvor oder im Folgenden bezüglich der Ausführungsbeispiele beschriebenen Merkmale der Erfindung. Insbesondere wird der Fachmann auch Einzelaspekte als Verbesserungen oder Ergänzungen zu der jeweiligen Grundform der vorliegenden Erfindung hinzufügen.

### INHALTSANGABE DER ZEICHNUNG.

Die vorliegende Erfindung wird nachfolgend anhand der in den schematischen Figuren der Zeichnungen angegebenen Ausführungsbeispiele näher erläutert. Es zeigen dabei:
- Fig. 1: eine schematische Ansicht einer Ausführungsform einer erfindungsgemäßen Kindersitzvorrichtung;
- Fig. 2: eine Perspektivansicht einer Ausführungsform der erfindungsgemäßen Kindersitzvorrichtung;
- Fig. 3: eine Ansicht eines Kinderwagens mit der erfindungsgemäßen Kindersitzvorrichtung gemäß Fig. 2;
- Fig. 4: eine Ansicht des Kinderwagens und der Kindersitzvorrichtung gemäß Fig. 3, wobei ein Kleinkind in der Kindersitzvorrichtung festgeschnallt ist.

Die beiliegenden Zeichnungen sollen ein weiteres Verständnis der Ausführungsformen der Erfindung vermitteln. Sie veranschaulichen Ausführungsformen und dienen im Zusammenhang mit der Beschreibung der Erklärung von Prinzipien und Konzepten der Erfindung. Andere Ausführungsformen und viele der genannten Vorteile ergeben sich im Hinblick auf die Zeichnungen. Die Elemente der Zeichnungen sind nicht notwendigerweise maßstabsgetreu zueinander gezeigt.

In den Figuren der Zeichnung sind gleiche, funktionsgleiche und gleich wirkende Elemente, Merkmale und Komponenten - sofern nichts Anderes ausführt ist - jeweils mit denselben Bezugszeichen versehen.

### BESCHREIBUNG VON AUSFÜHRUNGSBEISPIELEN

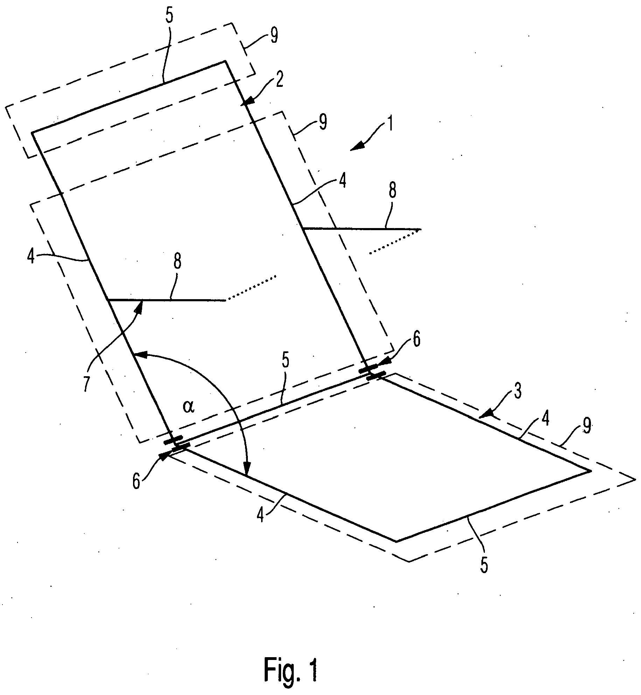

In Fig. 1 ist eine schematische, stark vereinfachte Darstellung einer Ausführungsform einer erfindungsgemäßen Kindersitzvorrichtung 1 gezeigt. Die Kindersitzvorrichtung 1 fungiert hier als Gesundheitssitz für Kinder zur Entwicklung einer gesunden Wirbelsäule.

Die erfindungsgemäße Kindersitzvorrichtung 1 kann insbesondere mit anderen Sitzeinrichtungen verwendet werden, wie z.B. Autositzen, Buggys und anderen Kindersitzen. Hierzu kann die Kindersitzvorrichtung 1 in dem Sitz, z.B. dem Autositz oder dem Sitz des Buggys, integriert sein oder als Einlage in dem jeweiligen Sitz angeordnet und wahlweise zusätzlich an diesem befestigt werden.

Die Kindersitzvorrichtung 1 weist, wie in Fig. 1 gezeigt ist, ein Sitzoberteil 2 oder Rückenteil und ein Sitzunterteil 3 oder Sitzteil auf. Das Sitzoberteil 2 und das Sitzunterteil 3 können fest miteinander verbunden sein und einen vorbestimmen, fest eingestellten Winkel α zueinander bilden. Ebenso können das Sitzoberteil 2 und das Sitzunterteil 3 auch derart schwenkbar miteinander verbunden sein, dass der Winkel α zwischen dem Sitzoberteil 2 und Sitzunterteil 3 variabel einstellbar ist.

Wie in dem Ausführungsbeispiel in Fig. 1 gezeigt ist, weisen das Sitzoberteil 2 und das Sitzunterteil 3 jeweils zwei Längsträger 4 auf. Die Längsträger 4 des Ober- und Sitzunterteils 2, 3 sind gemeinsam mit einem Querträger 5 verbunden, wobei das Sitzoberteil 2 und/oder das Sitzunterteil 3 um den Querträger 5 schwenkbar ausgebildet sind, beispielsweise mittels eines Scharniers. Das Sitzoberteil 2 und/oder das Sitzunterteil 3 bildet ein Scharnier 6 mit dem Querträger 5 zum Schwenken des Ober- bzw. Sitzunterteils 2, 3. Die Kindersitzvorrichtung 1 ist vorzugsweise mit einer Arretierung zur Arretierung des Sitzoberteils 2 und des Sitzunterteils 3 in verschiedenen Winkeln α zueinander versehen.

Zusätzlich können die Längsträger 4 des Sitzoberteils 2 bzw. des Sitzunterteils 3 zur weiteren Stabilisierung des Ober- bzw. Sitzunterteils 2, 3 jeweils mit wenigstens einem weiteren Querträger 5 verbunden sein. Beispielsweise kann ein Querträger 5 an einem oberen Ende oder einem Kopfbereich des Sitzoberteils 2 vorgesehen werden und mit den beiden Längsträgern 4 des Sitzoberteils 2 verbunden sein. Des Weiteren kann ein Längsträger 4 am unteren Ende des Sitzunterteils 3 vorgesehen werden und die beiden Längsträger 4 des Sitzunterteils 3 miteinander verbinden.

Die zwei Längsträger 4 des Sitzoberteils 2 bzw. die zwei Längsträger 4 des Sitzunterteils 3 können fest mit dem jeweiligen Querträger 5 verbunden werden, beispielsweise einteilig mit diesem ausgeformt sein oder durch Schweißen oder Löten usw. mit dem Querträger 5 verbunden sein. Ebenso können die beiden Längsträger 4 des Sitzoberteils 2 bzw. Sitzunterteils 3 auch lösbar mit dem zugeordneten Querträger 5 verbunden sein, beispielsweise durch Verschrauben und/oder Verstiften.

An dem Sitzoberteil 2 der Kindersitzvorrichtung 1 ist des Weiteren eine Stützeinrichtung 7 vorgesehen. Die Stützeinrichtung 7 weist auf beiden Seiten jeweils ein Stützelement 8 auf. Das Stützelement 8 kann fest mit dem Sitzoberteil 2 und dessen Längsträger 4 verbunden sein, beispielsweise einteilig mit dem Längsträger 4 ausgeformt sein oder durch Schweißen oder Löten mit diesem verbunden sein. Ebenso kann das Stützelement 8 auch entlang des Längsträgers 4 verschiebbar und somit höhenverstellbar vorgesehen sein. Das Stützelement 8 ist vorzugsweise in seiner jeweiligen Position arretierbar oder fixierbar an dem Längsträger 4 ausgebildet, um es in seiner eingestellten Höhe zu fixieren.

Die Stützelemente 8 können in der Höhe derart an dem Sitzoberteil 2 angebracht oder arretierbar sein, dass die Stützelemente 8 von hinten nach vorne den Oberkörper des Kindes auf der Ebene der Achselhöhle einfassen können. Auf diese Weise kann ein Teil des Körpergewichts des Kindes auf den Stützelementen 8 zum ruhen kommen. Andererseits können die seitlichen Bewegungsmöglichkeiten des Kindes eingeschränkt werden, wodurch ein ungewolltes seitliches Kippen der Wirbelsäule verhindert wird. Mit den Stützelementen 8 kann auch eine leichte Traktion oder ein leichter Zug auf den Oberkörper des Kindes realisiert werden, indem die Stützelemente 8 in einer geeigneten Höhe arretiert werden.

Die Stützelemente 8 sind, wie zuvor beschrieben, vorzugsweise höhenverstellbar ausgeführt, so dass sie an verschieden große Kinder angepasst werden können. Ebenso können die Stützelemente 8 optional auch ausziehbar ausgebildet sein (nicht dargestellt). Zudem sind die Stützelemente 8 bevorzugt an der Vorderseite mit einem verschließbaren Gurt (nicht dargestellt) versehen, der den sonst üblichen Gurtanschluss zwischen den Beinen des Kindes ersetzen kann.

Die Stützelemente 8 können je nach Funktion und Einsatzzweck eine beliebige Form aufweisen. Wie in der stark vereinfachten Darstellung in Fig. 1 gezeigt ist, können die Stützelemente 8 z.B. gerade oder beispielsweise gebogen oder abgewinkelt ausgebildet sein. Wie in Fig. 1 mit einer gepunkteten Linie angedeutet ist, können die Stützelemente 8 um den Körper des Kindes zumindest teilweise umgreifen.

Wie weiter in der schematischen Darstellung in Fig. 1 mit einer gestrichelten Linie angedeutet ist, ist die Kindersitzvorrichtung 1 vorzugsweise gepolstert oder zumindest mit einem Stoff oder einer Auflage bezogen. Dazu weist die Kindersitzvorrichtung 1 eine Polsterung des Sitzoberteils 2 und des Sitzunterteils 3 sowie wahlweise zusätzlich eine Polsterung (nicht dargestellt) der seitlichen Stützelemente 8 auf. Die Polsterung des Sitzoberteils 2 kann dabei ein z.B. durchgehendes Polsterteil oder zwei getrennte Polsterteile 9 aufweisen, d.h. ein Polsterteil 9 für den Rücken und ein Polsterteil 9 für den Kopf, wie in Fig. 1 gezeigt ist.

Die beiden Längsträger 4 der Kindersitzvorrichtung 1 sind jeweils ausziehbar ausgebildet. Dazu sind die Längsträger 4 beispielsweise zweiteilig ausgebildet, wobei ein erstes, unteres Längsträgerteil beispielsweise zur Aufnahme und Führung eines zweiten, oberen Längsträgerteils hohl ausgebildet ist. Das zweite Längsträgerteil kann ebenfalls hohl oder auch massiv ausgebildet sein. Das zweite Längsträgerteil ist in dem ersten Längsträgerteil verschiebbar angeordnet und zum Einstellen verschiedener Höhen des Polsterteils für den Kopf vorzugsweise in verschiedenen Positionen arretierbar oder fixierbar. Das Polsterteil 9 ist mit den beiden zweiten Längsträgerteilen der Längsträger 4 verbunden. Ein Querträger 5 kann die beiden zweiten Längsträgerteile miteinander verbinden und zusätzlich als Griff zum Ausziehen der beiden zweiten Längsträgerteile und damit zum Verlängern der Längsträger 4 dienen, vergleichbar einem ausziehbaren Griff bei einem Koffer. Das Polsterteil 9 für den Kopf ist ebenfalls an den beiden zweiten Längsträgerteilen befestigt und kann optional zusätzlich den Querträger 5 abdecken.

Die Kindersitzvorrichtung 1 gemäß der Erfindung ist zur Korrektur und Ausrichtung der Wirbelsäule oder des Rückrats und einer frühzeitigen Behandlung von Skoliose oder Wirbelsäulenverkrümmung vorgesehen. Bisher werden diese Korrekturen auf zwei Wegen durchgeführt. Ein Weg der Korrektur der Wirbelsäule ist das Einsetzen eines Implantats bei einem Erwachsenen oder einem Kind, wobei das Kind normalerweise älter als 16 Jahre ist, so dass die Ausbildung des Rückrats im Wesentlichen abgeschlossen ist. Der hierfür notwendige chirurgische Eingriff kann bei jüngeren Kindern nicht durchgeführt werden, deren Skelett sich noch in der aktiven Wachstumsphase befindet. Ein zweiter Weg, das Rückrat zu korrigieren, ist das Tragen eines orthopädischen Korsetts. Solche orthopädischen Korsetts weisen eine äußere Fixierung des Brustkorbs auf und sind infolgedessen hauptsächlich darauf ausgerichtet, dass eine vorhandene Verkrümmung der Wirbelsäule nicht weiter zunimmt oder sich verstärkt, ohne jedoch den Grund für die Verkrümmung zu behandeln. Das Tragen von orthopädischen Korsetts wird für Kinder im Alter von 13 bis 14 Jahren empfohlen. Ein Nachteil eines solchen orthopädischen Korsetts ist aber, dass es die Funktion der Muskulatur der Wirbelsäule einschränkt, was zu einer Unterfunktion und zu einer Athropie oder einem Schwinden der Muskulatur führt. Auch wenn die Verwendung eines orthopädischen Korsetts einen im Vergleich zum Einsetzen eines Implantats sehr viel geringfügigeren traumatischen Eingriff darstellt, so ist das orthopädische Korsett weitaus weniger wirksam wie ein Implantat.

Der Nachteil beider zuvor genannten Wege zur Behandlung einer Korrektur der Wirbelsäule besteht darin, dass diese bei sehr kleinen Kindern nicht eingesetzt werden können, da in diesem Alter sich das Skelett noch in einer sehr aktiven Wachstumsphase befindet. Ein Eingriff während dieses Wachstumsprozesses mit den beiden zuvor genannten Methoden kann die weitere Entwicklung der Wirbelsäule nachhaltig schädigen.

Gemäß der Erfindung wird daher eine Kindersitzvorrichtung 1 zur Behandlung einer Verkrümmung der Wirbelsäule bereitgestellt, welche die zuvor beschriebenen Probleme löst. Eine Korrektur der Verkrümmung der Wirbelsäule ist eminent wichtig, da eine Deformation oder Verkrümmung der Wirbelsäule im Kindesalter zu einer Vielzahl von Problemen im Erwachsenenalter führt. Die Erfindung stellt daher eine Kindersitzvorrichtung 1 bereit, welche ohne Operation auskommt und somit ohne traumatischen Eingriff der Ausbildung einer Wirbelsäulenverkrümmung vorbeugt. Außerdem ermöglicht die erfindungsgemäße Kindersitzvorrichtung 1 eine Korrektur einer bereits vorhandenen oder sich entwickelnden Wirbelsäulenverkrümmung oder Skoliose in einer sehr frühen Phase, nämlich im frühen Kindesalter, d.h. im Bereich von beispielsweise 6 bis 8 Monaten bis zu 2 bis 3 Jahren.

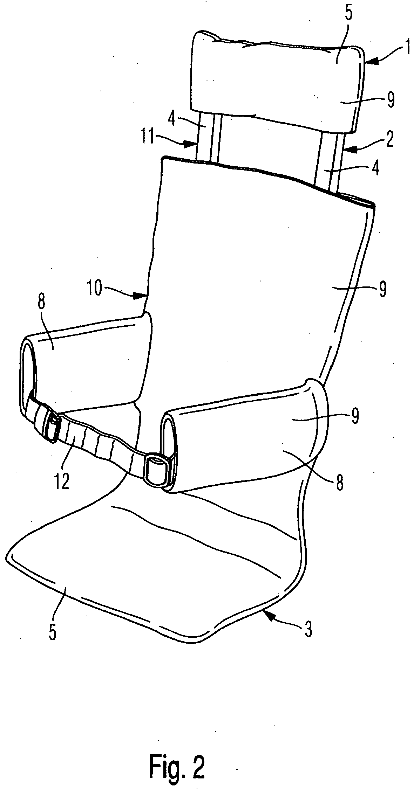

In Fig. 2 ist eine Perspektivansicht einer Ausführungsform der erfindungsgemäßen Kindersitzvorrichtung 1 gezeigt. Die erfindungsgemäße Kindersitzvorrichtung 1 kann als Sitz oder sogenannter traction seat fungieren, welcher den Körper eines Patienten leicht nach oben zieht und aufrichtet. Die Kindersitzvorrichtung 1 weist ein Sitzoberteil 2 und ein Sitzunterteil 3 auf, welche jeweils zwei Längsträger 4 aufweisen. Das Sitzoberteil 2 und das Sitzunterteil 3 sind beispielsweise über einen gemeinsamen Querträger 5 miteinander verbunden, wobei das Sitzoberteil 2 und/oder das Sitzunterteil 3 schwenkbar um den Querträger 5 ausgebildet ist, so dass verschiedene Winkel α zwischen dem Ober- und Sitzunterteil 2, 3 eingestellt werden können. Das Sitzoberteil 2 und/oder das Sitzunterteil 3 sind in dem jeweils eingestellten Winkel α arretierbar oder fixierbar ausgebildet.

Des Weiteren sind die beiden Längsträger 4 des Sitzoberteils 2 zusätzlich ausziehbar ausgebildet und in der ausgezogenen Position arretierbar. Wie zuvor mit Bezug auf Fig. 1 beschrieben, weist der jeweilige Längsträger 4 ein erstes Längsträgerteil 10 und ein zweites Längsträgerteil 11 auf, wobei das erste oder untere Längsträgerteil 10 zur Aufnahme und Führung des zweiten oder oberen Längsträgerteils 11 beispielsweise hohl ausgebildet ist. Das zweite Längsträgerteil 11 kann ebenfalls hohl oder aber auch massiv ausgebildet sein. Das zweite Längsträgerteil 11 ist in dem ersten Längsträgerteil 10 verschiebbar angeordnet und vorzugsweise in verschiedenen Positionen arretierbar oder fixierbar. Auf diese Weise können beispielsweise verschiedene Höhen eines Polsterteils 9 für den Kopf eingestellt werden.

Des Weiteren weist die in Fig. 2 gezeigte Ausführungsform der Kindersitzvorrichtung 1 zwei seitlich an den Längsträgern 4 angebrachte Stützelemente 8 auf, die von hinten nach vorne den Oberkörper des Kindes auf der Ebene der Achselhöhle einfassen und damit dessen seitliche Bewegung einschränken und vorzugsweise einen vorbestimmten oder definierten Zug auf das Kind, oberhalb der Brust des Kindes, aufbringen.

Die Stützelemente 8 können in der Höhe, also in vertikaler Richtung, verstellbar ausgebildet sein. Hierzu können die Stützelemente 8 verschiebbar an den Längsträgern 4 vorgesehen und vorzugsweise in verschiedenen Position oder Höhen arretierbar oder fixierbar ausgebildet sein.

Ebenso können die Stützelemente 8 zusätzlich oder alternativ auch seitlich oder in horizontaler Richtung der Kindersitzvorrichtung 1 einstellbar ausgebildet sein. Dazu können die Stützelemente 8 beispielsweise ausziehbar ausgebildet sein und vorzugsweise in der jeweils ausgezogenen Position arretierbar oder fixierbar sein.

Außerdem können die Stützelemente 8, das Sitzoberteil 2 und das Sitzunterteil 3 mit einer weichen Abdeckung oder Polsterung 9 versehen sein, wie in dem Ausführungsbeispiel in Fig. 2 gezeigt ist.

Die Stützelemente 8 sind auf der Vorderseite mit einem Gürtel 12 verbunden, um auf diese Weise eine Fixierung der Stützelemente 8 zwischen den Beinen des Kindes oder Babys zu vermeiden.

Der Boden des Sitzunterteils 3 der Kindersitzvorrichtung 1 ist flexibel ausgebildet und weist beispielsweise eine Polsterung auf, um, sofern erforderlich, das Kind oder Baby in einer horizontalen Position zu halten, ohne die Stützelemente 8 entfernen zu müssen.

Das Laufen eines Kindes in einer vertikalen Position wird durch das menschliche Gehirn gesteuert, insbesondere mit dem sogenannten Zerebellum oder Kleinhirn. Aus diesem Grund wird der Reflex für die vertikale bzw. aufrechte Position oder die gerade Haltung des Körpers durch den Aufrichter der Wirbelsäule (lat. musculus erector spinae oder musculus erector trunci) gesteuert. Aufgrund der physiologischen Asymmetrie der Gehirnhälften arbeiten solche Muskeln ebenfalls asymmetrisch. Da Muskelkrämpfe oder Muskelspasmen auf einer Seite stärker ausgeprägt sind, führt dies zu einer Neigung des Beckens zur gegenüberliegenden Seite. Die Beckenschieflage ist ein Grund für ein verkürztes rechtes oder linkes Bein mit der daraus folgenden Entwicklung von seitlichen Krümmungen der Wirbelsäule und einer Wirbelsäulenverkrümmung.

Wenn ein Kind zu laufen beginnt, wird die Position des Körpers Schritt für Schritt im Kleinhirn als sog. falscher Reflex der vertikalen Position des Körpers gespeichert und zwar für den Rest des Lebens. Es wurde nun herausgefunden, dass dieser Reflex, mit einem physiologisch richtigen Reflex ersetzt werden kann. Zu diesem Zweck wird, wenn das Kind selbständig zu stehen und zu laufen beginnt, eine korrigierende Einlage oder Einlegesohle unter die rechte oder linke Ferse gelegt, um das rechte oder linke Bein entsprechend anzuheben. Eine konstante Verwendung von korrigierenden Einlagen oder Einlegesohlen führt zu einer Wiederherstellung der symmetrischen Funktion des Aufrichters der Wirbelsäule (lat. musculus erector spinae oder musculus erector trunci), welcher aus mehreren Muskeln besteht. Dies führt dazu, dass keine Beckenschiefstellung auftritt, zudem hilft dies den Körper in einer aufrechten Position zu halten.

Die Erzeugung eines solchen neuen, physiologisch richtigen Reflexes kann jedoch früher erzeugt werden, wenn mit der Erzeugung dieses Reflexes begonnen wird, wenn die Eltern des Kindes beginnen, einen Buggy, oder Kinderwagen zu verwenden.

Die Stützelemente 8 der erfindungsgemäßen Kindersitzvorrichtung 1, welche den Körper in einer aufrechten Position halten, verhindern, dass dieser sich zur Seite biegen kann. Der Körper sendet außerdem Signale an das Kleinhirn zum Speichern oder Beibehalten dieser korrekten Position als einen normalen oder richtigen physiologischen Reflex. So entsteht, wenn das Kind zu laufen beginnt, keine Beckenschiefstellung und infolgedessen auch keine Ursache für das Ausbilden von Krümmungen der Wirbelsäule und einer Wirbelsäulenverkrümmung auf.

Außerdem kann die beschriebene Kindersitzvorrichtung 1 zur Korrektur der Wirbelsäule bei Kindern auch zum selben Zweck bei Erwachsenen, behinderten Personen oder geheingeschränkten Personen eingesetzt werden. Auch die Verwendung in einem Rollstuhl wäre denkbar. Die Idee einer solchen Korrektur liegt in einer leichten Zugwirkung auf den Körper und einer Minimierung einer seitlichen Bewegung des Körpers sowie einer Verhinderung des Biegens des Körpers zu einer Seite mit dem Stützelement.

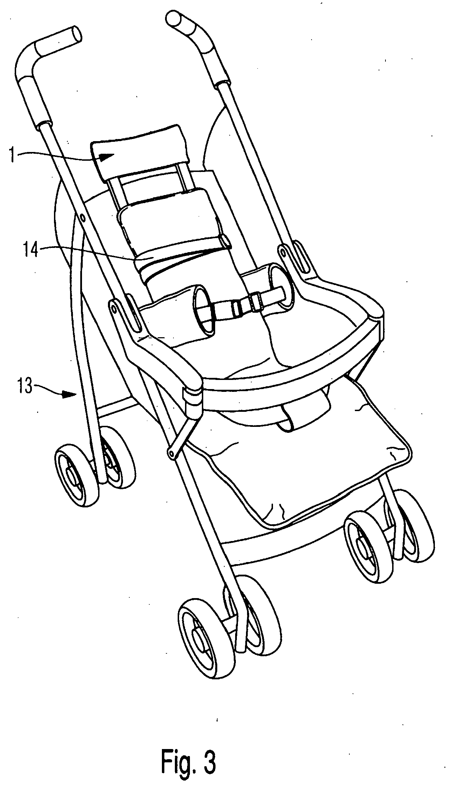

In Fig. 3 ist die Kindersitzvorrichtung 1 gemäß Fig. 2 gezeigt, wobei die Kindersitzvorrichtung 1 in einem Buggy als Beispiel für einen Kinderwagen 13 eingesetzt wird. In dem in Fig. 3 gezeigten Beispiel kann die Kindersitzvorrichtung 1 in dem Buggy 13 zusätzlich durch einen Gurt 14 des Buggy fixiert werden.

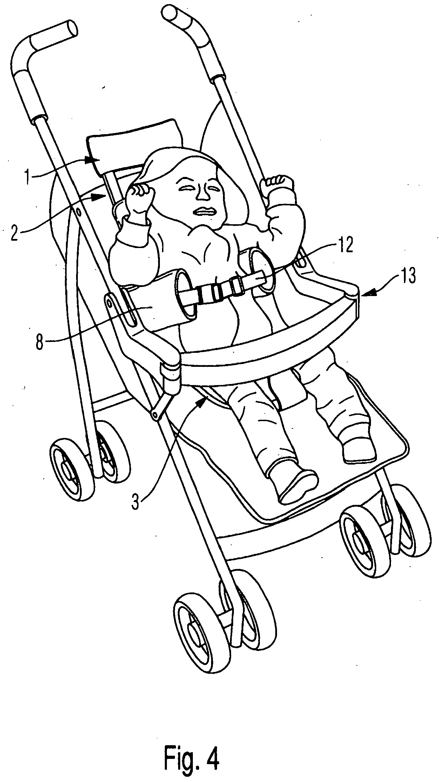

Fig. 4 zeigt den Buggy 13 mit der erfindungsgemäßen Kindersitzvorrichtung 1 aus Fig. 3, wobei ein Baby in der Kindersitzvorrichtung 1 sitzt. Hier fassen die Stützelemente 8 der Kindersitzvorrichtung 1 von hinten nach vorne den Oberkörper des Kindes auf der Ebene der Achselhöhle ein, so dass ein Teil des Körpergewichts des Kindes darauf zum Ruhen kommt. Des Weiteren schränken die Stützelemente 8 die seitliche Bewegungsmöglichkeit des Oberkörpers ein. Wie zuvor mit Bezug auf die Fig. 1 bis 3 beschrieben wurde, sind die Stützelemente 8 höhen- und/oder seitenverstellbar ausgeführt. Außerdem sind die Stützelemente 8 vorzugsweise an der Vorderseite mit einem verschließbaren Gurt 12 verbunden. Bei der Ausführung als Einlage in einen vorhandenen Sitz, wie hier einen Sitz eines Buggys 13, kann das Sitzunterteil 3 und/oder das Sitzoberteil 2 schwenkbar oder beweglich ausgebildet sein, beispielsweise mittels eines Scharniers, so dass bei einer Verstellung der Rückenlehne des Sitzes des Buggys 13 der eingelegte Sitz 1 entsprechen angepasst wird.

Obwohl die vorliegende Erfindung anhand bevorzugter Ausführungsbeispiele vorstehend vollständig beschrieben wurde, ist sie darauf nicht beschränkt, sondern auf vielfältige Art und Weise entsprechend den Ansprüchen modifizierbar.

### Bezugszeichenliste

- 1: Kindersitzvorrichtung
- 2: Sitzoberteil
- 3: Sitzunterteil
- 4: Längsträger
- 5: Querträger
- 6: Scharnier
- 7: Stützeinrichtung
- 8: Stützelement
- 9: Polster
- 10: erstes Längsträgerteil
- 11: zweites Längsträgerteil
- 12: Gurt (Kindersitzvorrichtung)
- 13: Kinderwagen
- 14: Gurt (Kinderwagen)

## Patentansprüche

1. Kindersitzvorrichtung (1) für ein Kind zur Ausrichtung der Wirbelsäule des Kindes und zur Vorbeugung und Behandlung einer Wirbelsäulenverkrümmung des Kindes (Skoliose), mit einem Sitzoberteil (2) und einem Sitzunterteil (3), wobei das Sitzoberteil (2) beidseitig mit jeweils einem Stützelement (8) versehen ist, welche derart ausgebildet und bezogen auf das Sitzoberteil (2) angeordnet sind, dass im Falle eines auf dem Kindersitz (1) sitzenden Kindes die Stützelemente (8) von hinten nach vorne den Brustkorb des Kindes auf der Ebene der Achselhöhle einfassen und damit dessen seitliche Bewegungen einschränken, **dadurch gekennzeichnet, dass** das Sitzoberteil (2) jeweils zwei Längsträger (4) aufweist, wobei die beiden Längsträger (4) ausziehbar ausgebildet und in der ausgezogenen Position arretierbar sind und wobei das Stützelement (8) fest mit dem Sitzoberteil (2) und dessen Längsträger (4) verbunden oder entlang des Längsträgers (4) verschiebbar und in seiner jeweiligen Position arretierbar an dem Längsträger (4) ausgebildet ist, um es in seiner eingestellten Höhe zu fixieren.

2. Kindersitzvorrichtung nach Anspruch 1,
**dadurch gekennzeichnet,**
**dass** die Stützelemente (8) höhenverstellbar und/oder seitlich verstellbar ausgebildet sind.

3. Kindersitzvorrichtung nach einem der vorstehenden Ansprüche,
**dadurch gekennzeichnet,**
**dass** eine Verschlussvorrichtung (12, 14) vorgesehen ist, über welche die Stützelemente (8) an ihrer Vorderseite verschließbar ausgebildet sind.

4. Kindersitzvorrichtung nach einem der vorstehenden Ansprüche,
**dadurch gekennzeichnet,**
**dass** ein Stützelement (8) gepolstert ausgebildet ist.

5. Kinersitzvorrichtung nach einem der vorstehenden Ansprüche,
**dadurch gekennzeichnet,**
**dass** ein Stützelemente (8) an dessen Oberseite, auf dem im bestimmungsgemäßen Gebrauch ein Arm des Kindes aufliegt, elastisch, abgerundet und/oder abgeflacht ausgebildet sind.

6. Kindersitzvorrichtung nach einem der vorstehenden Ansprüche,
**dadurch gekennzeichnet,**
**dass** zumindest ein federelastisches Element vorgesehen ist, über welches ein Stützelement (8) mit dem Sitzoberteil (2) federelastisch gekoppelt ist und welches dazu vorgesehen ist, bei vertikaler und/oder horizontaler Belastung des Stützelementes (8) ein federelastisches Nachgeben des Stützelementes (8) zu ermöglichen.

7. Kindersitzvorrichtung nach einem der vorstehenden Ansprüche,
**dadurch gekennzeichnet,**
**dass** das Sitzoberteil (2) und/oder das Sitzunterteil (3) schwenkbar ausgebildet ist und dass das Sitzoberteil (2) und/oder das Sitzunterteil (3) insbesondere in wenigstens einem vorbestimmten Winkel (α) zu dem Sitzunterteil (3) bzw. dem Sitzoberteil (2) arretierbar ausgebildet ist.

8. Kindersitzvorrichtung nach einem der vorstehenden Ansprüche,
**dadurch gekennzeichnet,**
**dass** das Sitzoberteil (2) ausziehbar ausgebildet ist.

9. Kindersitzvorrichtung nach einem der vorstehenden Ansprüche,
**dadurch gekennzeichnet,**
**dass** die Kindersitzvorrichtung (1) und insbesondere dessen Sitzoberteil (2), Sitzunterteil (3) und Stützelemente (8) derart dimensioniert und ausgebildet sind, um ein Kleinkind aufzunehmen.

10. Kindersitzvorrichtung nach einem der vorstehenden Ansprüche,
**dadurch gekennzeichnet,**
**dass** die Kindersitzvorrichtung (1) integraler Bestandteil des Kinderautositzes für ein Kraftfahrzeug ist oder die Kindersitzvorrichtung (1) als lösbarer Bestandteil in dem Kinderautositz vorgesehen oder befestigbar ist.

11. Kindersitzvorrichtung nach einem der vorstehenden Ansprüche,
**dadurch gekennzeichnet,**
**dass** die Stützelemente (8) den Oberkörper des Kindes auf der Ebene der Achselhöhle einfassen und damit dessen seitliche Bewegungen einschränken und einen Zug auf das Kind, oberhalb der Brust des Kindes, ausüben.

12. Kinderwagen (13), insbesondere Buggy,
mit einer Kindersitzvorrichtung (1) nach einem der Ansprüche 1 bis 11.

13. Kinderwagen nach Anspruch 12,
**dadurch gekennzeichnet,**
**dass** die Kindersitzvorrichtung (1) fester, integraler Bestandteil des Kinderwagens (13) ist.

14. Kinderwagen nach Anspruch 12,
**dadurch gekennzeichnet,**
**dass** die Kindersitzvorrichtung (1) als lösbarer Bestandteil in dem Kinderwagen (13) vorgesehen ist und über eine Befestigungseinrichtung an einem Rahmen des Kinderwagens (13) lösbar befestigbar ist.

15. Kinderautositz mit einer Kindersitzvorrichtung (1) nach einem der Ansprüche 1 bis 11.

## Claims

1. Child seat device (1) for a child for aligning the spinal column of the child and for preventing and treating a spinal column curvature (scoliosis) of the child,
comprising an upper seat part (2) and a lower seat part (3), wherein the upper seat part (2) is provided with a supporting element (8) on both sides, which supporting elements are designed and arranged relative to the upper seat part (2) in such a way that when a child is sitting on the child seat (1), the supporting elements (8) surround the chest of the child from the rear to the front at armpit level and thus limit the lateral movements of the child,
**characterised in that** the upper seat part (2) comprises two longitudinal supports (4) in each case, wherein the two longitudinal supports (4) are extendible and can be locked in the extended position, and wherein the supporting element (8) is fixedly connected to the upper seat part (2) and the longitudinal support (4) thereof or is formed on the longitudinal support (4) to be displaceable along the longitudinal support (4) and lockable in its respective position, in order to fix it at its adjusted height.

2. Child seat device as claimed in claim 1, **characterised in that** the supporting elements (8) are height-adjustable and/or laterally adjustable.

3. Child seat device as claimed in any one of the preceding claims, **characterised in that** a locking device (12, 14) is provided via which the supporting elements (8) are lockable on their front face.

4. Child seat device as claimed in any one of the preceding claims, **characterised in that** a supporting element (8) is padded.

5. Child seat device as claimed in any one of the preceding claims, **characterised in that** a supporting element (8) is formed in an elastic, rounded and/or flattened manner on its upper face against which an arm of the child lies when the device is used as intended.

6. Child seat device as claimed in any one of the preceding claims, **characterised in that** at least one resilient element is provided via which a supporting element (8) is coupled to the upper seat part (2) in a resilient manner and which is provided to permit resilient yielding of the supporting element (8) when the supporting element (8) is loaded vertically and/or horizontally.

7. Child seat device as claimed in any one of the preceding claims, **characterised in that** the upper seat part (2) and/or the lower seat part (3) are pivotable, and **in that** the upper seat part (2) and/or the lower seat part (3) are lockable in particular at at least one predetermined angle (α) with respect to the lower seat part (3) and the upper seat part (2), respectively.

8. Child seat device as claimed in any one of the preceding claims, **characterised in that** the upper seat part (2) is extendible.

9. Child seat device as claimed in any one of the preceding claims, **characterised in that** the child seat device (1) and in particular its upper seat part (2), lower seat part (3) and supporting elements (8) are dimensioned and formed so as to accommodate an infant.

10. Child seat device as claimed in any one of the preceding claims, **characterised in that** the child seat device (1) is an integral component of the child car seat for a motor vehicle or the child seat device (1) is provided or can be attached as a releasable component in the child car seat.

11. Child seat device as claimed in any one of the preceding claims, **characterised in that** the supporting elements (8) surround the upper body of the child at armpit level and therefore limit its lateral movements and exert a pull on the child, above the chest of the child.

12. Stroller (13), in particular a buggy, comprising a child seat device (1) as claimed in any one of claims 1 to 11.

13. Stroller as claimed in claim 12, **characterised in that** the child seat device (1) is a fixed integral component of the stroller (13).

14. Stroller as claimed in claim 12, **characterised in that** the child seat device (1) is provided as a releasable component in the stroller (13) and can be releasably attached to a frame of the stroller (13) by means of an attachment device.

15. Child car seat comprising a child seat device (1) as claimed in any one of claims 1 to 11.

## Revendications

1. Dispositif de siège enfant (1) destiné à un enfant, pour l'orientation de la colonne vertébrale de l'enfant et pour la prévention et le traitement de la scoliose de l'enfant,
comportant une partie supérieure de siège (2) et une partie inférieure de siège (3), la partie supérieure de siège (2) étant munie respectivement d'un élément de support (8) sur chaque côté, ceux-ci étant réalisés et disposés de telle manière par rapport à la partie supérieure de siège (2) que, lorsqu'un enfant est assis sur le siège enfant (1), les éléments de support (8) entourent de l'arrière vers l'avant la cage thoracique de l'enfant au niveau des aisselles et limitent ainsi son mouvement latéral,
**caractérisé en ce que** la partie supérieure de siège (2) présente respectivement deux supports longitudinaux (4), les deux supports longitudinaux (4) étant réalisés extensibles et pouvant être bloqués dans la position sortie et l'élément de support (8) étant relié solidairement à la partie supérieure de siège (2) et à son support longitudinal (4) ou réalisé coulissant le long du support longitudinal (4) et bloqué dans sa position respective au niveau du support longitudinal (4), pour le fixer à sa hauteur réglée.

2. Dispositif de siège enfant selon la revendication 1, **caractérisé en ce que** les éléments de support (8) sont réalisés réglables en hauteur et/ou réglables latéralement.

3. Dispositif de siège enfant selon l'une des revendications précédentes, **caractérisé en ce qu'**un dispositif de fermeture (12, 14) est prévu, par l'intermédiaire duquel les éléments de support (8) sont réalisés refermables au niveau de leur partie avant.

4. Dispositif de siège enfant selon l'une des revendications précédentes, **caractérisé en ce qu'**un élément de support (8) est réalisé rembourré.

5. Dispositif de siège enfant selon l'une des revendications précédentes, **caractérisé en ce qu'**un élément de support (8) est réalisé élastique, arrondi et/ou aplati au niveau de la partie supérieure de celui-ci sur lequel repose un bras de l'enfant lors d'un usage approprié.

6. Dispositif de siège enfant selon l'une des revendications précédentes, **caractérisé en ce qu'**au moins un élément à effet de ressort est prévu, par l'intermédiaire duquel un élément de support (8) est accouplé en élasticité de ressort à la partie supérieure de siège (2) et qui est prévu pour permettre en cas de charge verticale et/ou horizontale de l'élément de support (8) un fléchissement à effet de ressort de l'élément de support (8).

7. Dispositif de siège enfant selon l'une des revendications précédentes, **caractérisé en ce que** la partie supérieure de siège (2) et/ou la partie inférieure de siège (3) est réalisée pivotante et que la partie supérieure de siège (2) et/ou la partie inférieure de siège (3) est réalisée de manière à pouvoir être bloquée en particulier selon au moins un angle (α) prédéterminé par rapport à la partie inférieure de siège (3) ou de la partie supérieure de siège (2).

8. Dispositif de siège enfant selon l'une des revendications précédentes, **caractérisé en ce que** la partie supérieure de siège (2) est réalisée extensible.

9. Dispositif de siège enfant selon l'une des revendications précédentes, **caractérisé en ce que** le dispositif de siège enfant (1) et en particulier sa partie supérieure de siège (2), sa partie inférieure de siège (3) et ses éléments de support (8) sont dimensionnés et réalisés de manière à loger un petit enfant.

10. Dispositif de siège enfant selon l'une des revendications précédentes, **caractérisé en ce que** le dispositif de siège enfant (1) fait partie intégrante du siège de véhicule pour enfant pour un véhicule ou le dispositif de siège enfant (1) est prévu comme une partie amovible ou peut être fixé dans le siège de véhicule pour enfant.

11. Dispositif de siège enfant selon l'une des revendications précédentes, **caractérisé en ce que** les éléments de support (8) bordent le haut du corps de l'enfant au niveau des aisselles et limitent ainsi ses mouvements latéraux et exercent une traction sur l'enfant, au-dessus de la poitrine de l'enfant.

12. Poussette (13), en particulier poussette-canne, comportant un dispositif de siège enfant (1) selon l'une des revendications 1 à 11.

13. Poussette selon la revendication 12, **caractérisée en ce que** le dispositif de siège enfant (1) est une partie intégrante et fixe de la poussette (13).

14. Poussette selon la revendication 12, **caractérisée en ce que** le dispositif de siège enfant (1) est prévu comme une partie amovible dans la poussette (13) et peut être fixé de manière amovible par l'intermédiaire d'un moyen de fixation au niveau d'un châssis de la poussette (13).

15. Siège de véhicule pour enfant avec un dispositif de siège enfant (1) selon l'une des revendications 1 à 11.
